# EUROPEAN PATENT SPECIFICATION

(11) **EP 2 869 787 B1**
(45) Date of publication and mention of the grant of the patent: **19.09.2018**
(21) Application number: 13725208.6
(22) Date of filing: 08.04.2013
(51) Int. Cl.: A61F 2/28, B29C 45/00, F28F 13/00

(54) **3D POROUS MOULDED-MULTILAYER PRODUCT AND PRODUCTION METHOD THEREOF**
PORÖSES MEHRSCHICHTIGES 3D-FORMPRODUKT UND HERSTELLUNGSVERFAHREN DAFÜR
PRODUIT MULTICOUCHE MOULÉ POREUX EN TROIS DIMENSIONS ET SON PROCÉDÉ DE PRODUCTION

(30) Priority: 04.07.2012 PT 12106428 U
(43) Date of publication of application: 13.05.2015
(73) Proprietor: Celoplas - Plásticos Para A Industria S.A., 4775-127 Grimancelos, Barcelos (PT)
(72) Inventor: CUNHA REIS RODRIGUES SAMPAIO, Marcos, P-4715-343 Braga (PT); DE OLIVEIRA CORTEZ, João, P-4760-221 Vila Nova de Famalicão (PT)
(74) Representative: Teixeira de Carvalho, Anabela
(86) International application number: PCT/IB2013/052796
(87) International publication number: WO 2014/006519

(56) References cited:
- FR-A1- 2 580 794
- US-A- 5 348 788
- US-A1- 2006 241 776
- US-A1- 2006 241 776
- US-A1- 2009 061 127

## Description

### Technical field

The disclosure relates to the production of a bone implant comprising three-dimensional porous structures comprising multiple moulded layer modules with interconnected channels in all 3D substantially axial and/or radial directions namely for medical applications.

### Background

Three-dimensional porous structures with interconnected channels are made through three leading methods. These are foaming, foam replication, and solid free forming, or 3D printing, processes.

In foaming or replication processes, non-uniform structures are achieved. The geometry of the channels is dictated by the formation, growth and impingement of gaseous cells (pore). In general, pores are spherical in shape. Level of pores interconnectivity is only possible to be determined with special techniques, and is highly influenced by process parameters.

In 3D printing processes or solid free forming processes, three-dimensional porous structures are produced by deposition of materials, layer by layer, using one or several nozzles at once. These methods allow the manufacture of exclusive parts, but are still slow and expensive for mass production.

Document FR2580794A1 discloses a heat exchange device whose exchange zone is formed by juxtaposing a plurality of plates parallel with each other and spaced apart two by two by a plurality of spacing elements consisting of spacers parallel to each other, which may be crenellated (they then comprise crenels and projecting parts) or continuous, said plates being formed so that for any two adjacent plates, at lest one of the facing faces is provided with spacers, at least one part of said spacers being, over at least a part of their length, in contact with the facing face of the adjacent plate or with at least a part of the spacers possibly present on said facing face, over at least a part of their length, said plates, defining flow spaces for two fluids being further formed and disposed so that for one flow space out of two the spacers carried by at least one of the facing faces of two adjacent plates defining said space are crenellated spacers. The fluids flow advantageous in cross-current relation. The heat exchange devices thus formed, whose plates may be obtained readily by molding (light alloys, thermoplastic materials or thermosetting materials) are advantageously used for exchanges between gases, for example for recovering heat from the smoke of boilers or furnaces.

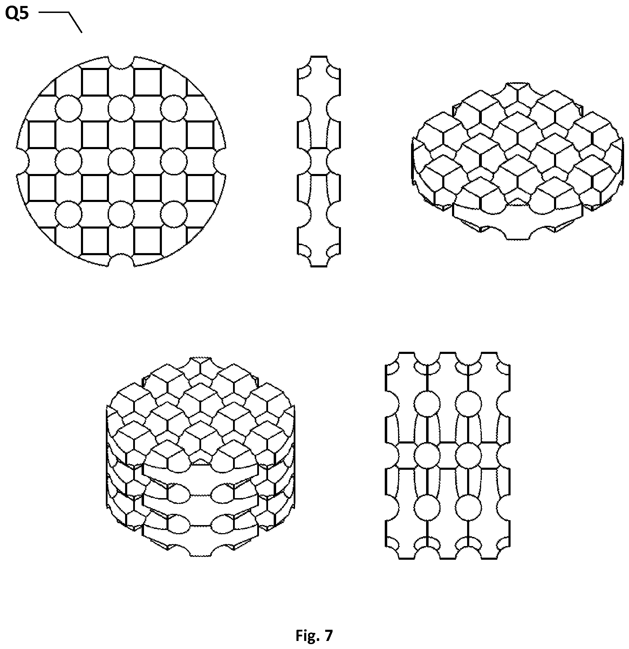

Patent document US2009/0061127A1 (Entezarian et al) describes the manufacture of porous structures through injection moulding. In this document, a very complex injection moulding tool is described in which several straight elements are organized in a way that allows the manufacture in a single step of simple geometric structures with interconnected channels. The section of the channels is defined by the section of the pins that are organized and aligned in two different directions. The channel in the third direction is obtained as a consequence of the tangency of the previous described inserts (e.g. Fig. 7B). For this reason, the interconnectivity section of the channels is limited by the tangent surface of the moulding pins.

Other prior art, namely described in document EP0672395A1 (Salvatore et al) describes a method specially conceived for the production of medical application parts in order to improve osteointegration. This method allows the production of complex shape parts in a system of layer-by-layer powder deposition, allowing the production of complex free form parts, dispensing the use of a mold to conform the materials. However, this system does not allow the mass production of parts in a systematic approach, since it is based in a slow method of individual part production, that need to be previously generated in a Computer Assisted Design system.

The method claimed in the above cited document EP0672395A1 is suited for individually specific parts, with precise properties and does not allow the easy flexibility of form and chain production ability of the described invention. Moreover, said method has strong limitation on the type of materials that can be used to produce the final part, being dependent on the powder suspension formulations available for the technology. Thus, document EP0672395A1 cannot be reduced to practice using a wide range of materials.

Document EP0647439A2 refers the use of layer modules to obtain three-dimensional porous moulded-multilayer products. Its layers are symmetrical in the Z axis and require two subsequently reversed layers for channels in all 3D directions to be formed. Thus, it requires a substantial amount of joints between layers, which increases the probability of layer adhesion problems and, thus, structural faults. It also requires that subsequent layers must be 'turned upside down' and the complexity of turning such small-scale objects is considerable. Furthermore, for aligning protrusions with protrusions, the alignment of the layers before joining must be rather precise, with corresponding higher costs. In fact, the disclosure does not seem enabling as a mandrel is mentioned for recovering the alignment between through-holes of different layers, which is neither practically feasible nor economic, depending on the through-hole diameter. This document also refers that the layers are stacked by rolling up multiple times a sheet layer - but as different roll layers have different roll perimeters, the through-holes will only align infrequently and thus through-holes will not be formed through the material.

These facts are disclosed in order to illustrate the technical problem addressed by the present disclosure.

### Summary

It is disclosed a bone-implant comprising a three-dimensional porous moulded-multilayer product (2) comprising a stack assembly of a plurality of injection moulded layer modules (1), said modules (1) comprising:
a plurality of through-holes between the layer surfaces of said moulded layer module (1);
a plurality of longitudinal recesses on at least one of the layer surfaces of said moulded layer module (1), along at least two different directions, such that when assembled with a layer surface of another moulded layer module (1), longitudinal
channels are formed;
wherein said through-holes and recess channels are interconnected.

In an embodiment, the plurality of modules (1) is assembled with all layer faces of said modules (1) positioned in the same direction.

In an embodiment, said module (1) comprises alignment features on both layer surfaces of said moulded layer module (1), in particular protrusions and recesses for receiving protrusions, said features being such that, when a moulded layer module is assembled with another moulded layer module, a partial or full overlap of the through-holes of said moulded layer modules is obtained.

In an embodiment, the moulded layer module (1) is obtainable by powder injection moulding, thermoplastic injection, powder pressing or machining, or combinations thereof.

In an embodiment, the moulded layer module (1) comprises plastic, thermoplastic polymer, thermoset resin, metal, or ceramic, or combinations thereof.

In an embodiment, the moulded layer module (1) comprises bioresorbable polymer, bioresorbable ceramic, bio-inert polymer, bio-inert ceramic, bio-inert metallic, or heat-conductive material, or combinations thereof.

In an embodiment, the cross-section of the through-holes and longitudinal channels of the moulded layer module (1) is hexagonal (Q1), square (Q2, Q7), circular (Q3, Q5, Q8), and/or cross-shaped (Q4).

In an embodiment, the through-holes of the of the moulded layer module (1) are laid out in a grid (Q1, Q2, Q4, Q5, Q6), or concentrically (Q3, Q8), or both in a grid and concentrically (Q7).

In an embodiment, the through-holes of the moulded layer module (1) are perpendicular to the layer surfaces of said moulded layer module (1) or are tilted relative to the layer surfaces of said moulded layer module (1).

It is also disclosed that the moulded-multilayer products (Q1, Q2, Q3, Q4, Q5, Q6, Q7, Q8, Q9 and Q10) are aligned based on the alignment features principles represented in (Q11, Q12). The referred features are added to the design definition of the moulded layer module (1) to guaranty the alignment and precision of the moulded-multilayer products (2). Controlling the alignment between layers is relevant for controlling the overall porosity of the product. It should be noted that this porosity is not strictly porosity in the sense of the proportion between voids and solid in the product. For example, for numerous applications, porosity is better evaluated as effective porosity, i.e. not considering voids that are not interconnected. Furthermore, for example, for biocompatibility, the interconnections must be reasonably accessible, i.e. relatively straight, or with a precisely controllable meandering aspect. Thus the importance of controlling the alignment of the layers and thus the overall product characteristics.

In an embodiment, the three-dimensional porous moulded-multilayer product (2) assembly is obtainable by sintering, welding, ultrasonic welding, rigid junctions, screws, spikes, rivets or snap fits, or combinations thereof.

In an embodiment, the assembled plurality of moulded layer modules (1) of the three-dimensional porous moulded-multilayer product (2) is offset between adjacent moulded layer modules (1), in particular such that adjacent moulded layer modules (1) are translationally, rotationally or randomly offset, in order that adjacent moulded layer modules (1) through-holes are partially overlapping.

In an embodiment, the three-dimensional porous moulded-multilayer product (2) is for use in medicine.

In an embodiment, the three-dimensional porous moulded-multilayer product (2) is for use in bone implants.

It is also disclosed a mould for the moulded layer module (1) according to any one of the previous embodiments comprising the inverse features for obtaining said moulded layer module (1) features.

It is also disclosed a method for producing a bone implant comprising a three-dimensional porous moulded-multilayer product (2) comprising the steps of:
injection moulding a plurality of moulded layer modules (1) according to any of the previously described;
assembling said plurality of moulded layer modules (1) in a stack.

In an embodiment, the moulding of the moulded layer module (1) comprises powder injection moulding, thermoplastic injection moulding, powder pressing or machining, or combinations thereof.

In an embodiment, the assembling of the moulded layer modules (1) comprises applying rigid junctions, screws, spikes, rivets or snap fits, sintering, welding, ultrasonic welding, or combinations thereof.

In an embodiment, the method further comprises, in the moulding step of the moulded layer modules (1), moulding with a binder, and further comprising, before or during the assembly step, removing said binder, in particular thermally removing said binder during the assembly operation, said assembly operation by sintering.

In an embodiment, said binder is polymeric and the removal of said binder is thermal, catalytic or by solubility.

The disclosure relates to the production of a bone implant comprising three-dimensional porous structures comprising multiple moulded layer modules with interconnected channels in all 3D substantially axial and/or radial directions. The directions may be orthogonal or may be not orthogonal but with also 3 different directions obtained in a 3D space. The two directions in each layer may be perpendicular or not. The through-holes, when considered from the through-hole axis, may be perpendicular to each layer, may be perpendicular to a part of the layers, or may not be perpendicular to any layer.

One of the objects of the disclosure is a high porosity three-dimensional multi-layer, or multi-module, device comprising interconnected three-dimensional channels and controlled (geometric and dimensional) cross-sections (constant or variable) obtained by the assembly of several modular parts and the process to produce said device namely for medical applications.

The modular or layer parts can be plastic, metallic, ceramic or any compound comprising some and/or all the cited materials and obtained through moulding techniques, namely powder injection moulding (metallic or ceramic materials), thermoplastic injection, powder pressing or machining techniques.

The modular or layer parts include design features to allow the assembly alignment (Q11, Q12). These features will guaranty the channel concentricity and, for example, consequent improved biocompatibility in orthopaedic applications.

The final devices may be assembled by different techniques namely by powder sintering techniques, ultrasonic welding, rigid junctions (screws, spiking, rivets), snap fits in order to obtain multiple product shapes.

The combination of any of the previous described techniques may also be used.

### Brief Description of the Drawings

The following figures provide preferred embodiments for illustrating the description and should not be seen as limiting the scope of invention.
**Fig. 1****:** Schematic representation of the coordinate system which serves as reference for the disclosure.
**Fig. 2****:** Schematic representation of the assembly of three layer modules (1) into an assembled three-dimensional porous structure comprising interconnected channels in all 3D axial directions.
**Fig. 3****:** Schematic representation of a layer module, and respective assembled 3D porous structure comprising interconnected channels in all 3D axial directions, with hexagonal cross-section (Q1).
**Fig. 4****:** Schematic representation of a layer module, and respective assembled 3D porous structure comprising interconnected channels in all 3D axial directions, with square cross-section (Q2). The channel cross-sections are formed 50% in one of the layer module XY surfaces being the other 50% formed through the opposite XY surface of the same layer module.
**Fig. 5****:** Schematic representation of a layer module, and respective assembled 3D porous structure comprising interconnected channels in all 3D cylindrical directions (longitudinal, radial, concentric), with circular cross-section (Q3).
**Fig. 6****:** Schematic representation of a layer module, and respective assembled 3D porous structure comprising interconnected channels in all 3D axial directions, with cross-shaped cross-section (Q4).
**Fig. 7****:** Schematic representation of a layer module, and respective assembled 3D porous structure comprising interconnected channels in all 3D axial directions, with circular cross-section (Q5).
**Fig. 8****:** Schematic representation of a layer module, and respective assembled 3D porous structure comprising interconnected channels in all 3D axial directions, with X-Y square cross-section and non-rectilinear centreline and distorted Z square cross-section and rectilinear centreline (Q6).
**Fig. 9****:** Schematic representation of a layer module, and respective assembled 3D porous structure comprising interconnected channels in all 3D axial directions, with square cross-section and further channels at both 45 degrees relative to the X-Y axis (Q7).
**Fig. 10****:** Schematic representation of a layer module, and respective assembled 3D porous structure comprising interconnected channels in all 3D cylindrical directions (longitudinal, radial, concentric), with circular cross-section which increases towards the longitudinal centre of the assembly (Q8).
**Fig. 11****:** Schematic representation of the moulded-multilayer product (2) with channels slanted along the Z axis. a) The moulded-multilayer module has slated channels with the Z axis. b) The moulded-multilayer module has channels parallel to the Z axis. In both cases, the channel continuity along the Z axis can be obtained by translations (in the XY plan), rotations (along the Z axis) or both of the previous movements of the different moulded multilayer modules.

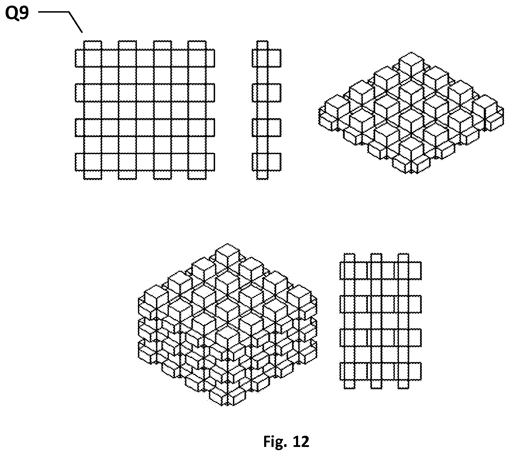

**Fig. 12****:** Schematic representation of a layer module and respective assembled 3D porous structure comprising interconnected channels in all 3D axial directions with square cross-sections (Q9). The channel cross-sections are formed 25% in one of the layer module XY surfaces being the other 75% formed through the opposite XY surface of the same layer module.

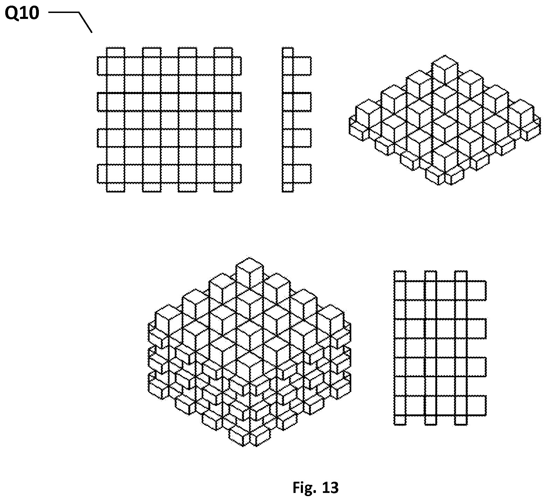

**Fig. 13****:** Schematic representation of a layer module and respective assembled 3D porous structure comprising interconnected channels in all 3D axial directions with square cross-sections (Q10). The channels cross-sections are formed 100% in one of the layer module XY surface.

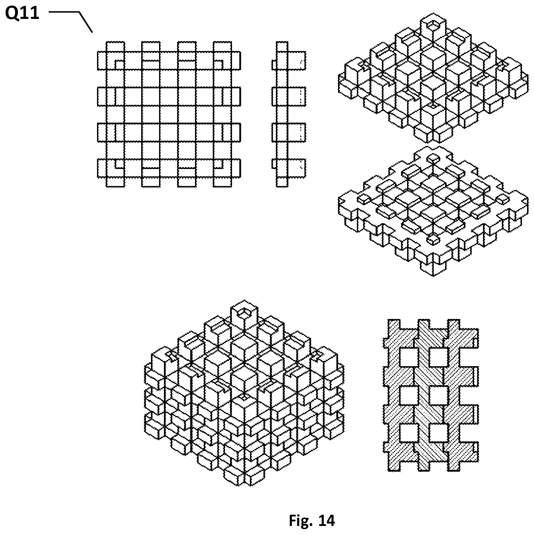

**Fig. 14****:** Schematic representation of a layer module and respective 3D porous structure comprising interconnected channels in all 3D axial directions with square cross-sections and XY alignment features (Q11). The channel cross-sections as well as the alignment features are formed 50% in one of the layer module XY surfaces being the other 50% formed through the opposite XY surface of the same layer module.

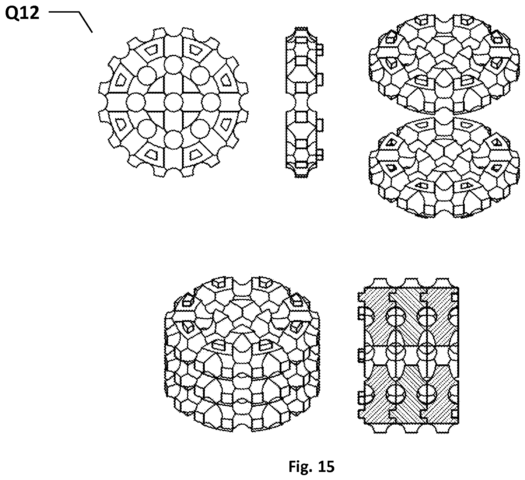

**Fig. 15****:** Schematic representation of a layer module and respective 3D porous structure comprising interconnected channels in all 3D cylindrical directions (longitudinal, radial, and concentric) with circular cross-sections and XY alignment features (Q12). The channel cross-sections as well as the alignment features are formed 50% in one of the layer module XY surfaces being the other 50% formed through the opposite XY surface of the same layer module.

The devices represented in each figure are obtained by the replicated use of the respective modules.

In some embodiments, combinations of different modules may be used to create a complex multi-shape part or device.

The previous represented devices are fully interconnected. Some embodiments may be partially interconnected to allow for example the creation of customized and precise channels or tracks.

The represented Figures consider that the channels in the plan defined by the XY axis (surface contact plan between modules) are generally defined 50% in the upper surface and 50% in the bottom surface (upper and lower modules). Nevertheless, some embodiments can be customized in a range from 0% up to 100% (Q9, Q10) in each of the referred surfaces (upper and lower modules). In the case of 100% (Q10) of the channel being in one of the layer module surfaces, then no channel is required on the opposite side of the layer module surface simplifying design and construction. However, if the channel has a convex cross-section, namely circular or hexagonal, it may be preferable to provide part of the channel in both surfaces of the layer module such that ejection from the mould can be facilitated.

The represented Figures consider the assembly of the several modules only along the Z-axis.

Some embodiments can also consider the assembly in any direction over the plan defined by the XY axis. For example, previously assembled layer modules Q1, Q2 and Q4 may be easily assembled laterally with other similar previously assembled layer modules.

Slanted assemblies are also possible where the stack assembly angle is not at perpendicular angles to the layer surfaces of the layer module.

### Detailed Description of the Invention

The disclosure comprises a bone implant comprising a high porosity three-dimensional multi-layer device comprising interconnected three-dimensional channels and controlled (geometric and dimensional) cross-sections (constant or variable) obtained by the assembly of several modular parts and the processes to produce said device namely for medical applications.

For medical applications, such devices may be used as bone implants, taking in consideration the exceptional condition provided by the high exposed area, which will facilitate the bone implant absorption and osteointegration. Moreover the ability to conceive and produce any shape in mass production represents a huge advance in the approach that can be used to setup new standard shapes and models for this field of application.

The disclosure is generally suited for all applications with the requirement to maximize the exposed area of a material to increase the functionality provided by the surface area, or whenever the internal shape tends to be very complex (like multichannel of variable design) which might limit the final shape design of the parts, due to the production technique constraints.

The materials to be used can be any mouldable or machined material, namely plastic, metallic, ceramic or any compound comprising some and/or all the cited materials.

In some embodiments, the material includes one or more of a thermoplastic polymer, a thermoset resin, a ceramic or a metal. Metals or ceramics can be supplied in a polymer matrix solution, namely powder injection moulding feedstock's (market available or customized for any particular use and/or embodiment).

In some embodiments, the material can include (at least partially, more than half, substantially, or essentially) a biocompatible material such as a bioresorbable polymer or a bioresorbable ceramic. In some of these embodiments, the material may comprise a bio-inert polymer, a bio-inert ceramic, or a bio-inert metallic.

In some embodiments, the material may comprise a heat-conductive material.

The modular parts produced may have porosity (% of voids / total volume) from 10% up to 90% approximately. In some embodiments, the porosity can be less than 10% or higher than 90%. The porosity mentioned above relates to the spaces created by the channels and through holes of the product modular layer design, regardless of (or non-conditioned by) the intrinsic porosity of the raw material used on for device production.

Channels in the structure may take any cross-sectional forms or shapes. Said channels may be squared, circular, triangular, trapezoidal or any other free form shapes resulting from the combination of the previous ones. Examples of possible combinations are represented in the figures. In some embodiments, it is advantageous if the cross-sectional shape is the same for channels formed by the recesses in the surface of the layer modules as the cross-sectional shape of the through-holes of the layer modules.

One, some, substantially all, or essentially all of the channels created in the structure may have a cross-sectional area of at least about 0.001mm², up to about 100mm². In some embodiments, the cross-sectional area may be less than 0.001 mm², or greater than 100mm².

Depending on the embodiment, the channels may have a cross-sectional area of at least about 0.002 square millimetres, at least about 0.01 square millimetres, at least about 0.25 square millimetres, at least about 0.5 square millimetres, at least about 1 square millimetre, at least about 2.25 square millimetre, at least about 4 square millimetre, at least about 9 square millimetre, at least about 16 square millimetre, at least about 25 square millimetre, up to about 100 square millimetres, up to about 25 square millimetre, up to about 16 square millimetres, up to about 9 square millimetres, up to about 4 square millimetre, up to about 2.25 square millimetre, up to about 1 square millimetre, up to about 0.5 square millimetre, up to about 0.25 square millimetre, and/or up to about 0.01 square millimetre. In some embodiments, the cross-sectional area may be less than 0.002 square millimetres or greater than 100 square millimetres.

Depending on the embodiment, the channels may have a cross-sectional diameter (i.e. the average length between internal sides of a pore measured from a two-dimensional cross-section of the pore, the cross-section being perpendicular to the direction in which the pore extends) of up to about 7 mm, up to about 4 mm, up to about 3 mm, up to about 2 mm, up to about 1500 microns, up to about 800 microns, up to about 400 microns, up to about 200 microns, up to about 150 microns, up to about 125 microns, up to about 100 microns, up to about 80 microns, up to about 50 microns, at least about 20 microns, at least about 50 microns, at least about 100 microns, at least about 150 microns, at least about 200 microns, at least about 300 microns, at least about 400 microns, at least about 600 microns, at least about 1 mm, and/or at least about 2 mm.

In some embodiments, for osteointegration embodiments, being able to define the channel dimensions is of particular interest.

In some embodiments, the cross section can assume a non-uniform diameter. In these cases, the average cross-sectional diameter can be measured, for example, by picking a point in the centre of the channel and measuring (and averaging) the length of a line extending between internal sides of the channel at different angles, say every 5 degrees (i.e. rotating the lines by 5 degrees for on every measurement).

In some embodiments, the width of the channels may vary within the same structure, as it is not necessary that these have all the same diameter or cross-sectional area. In an embodiment, at least 70% of the channels that are larger than the average channel size of the structure, have a cross-sectional area that is less than 10 times the average channel size of the structure.

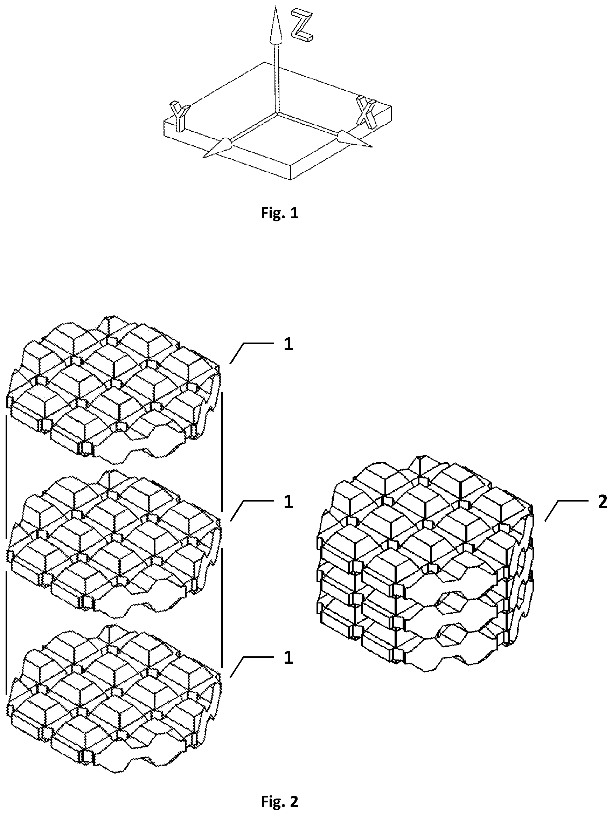

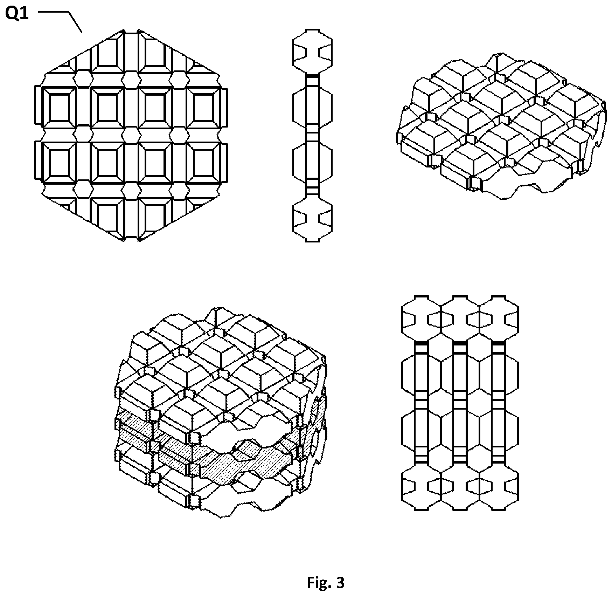

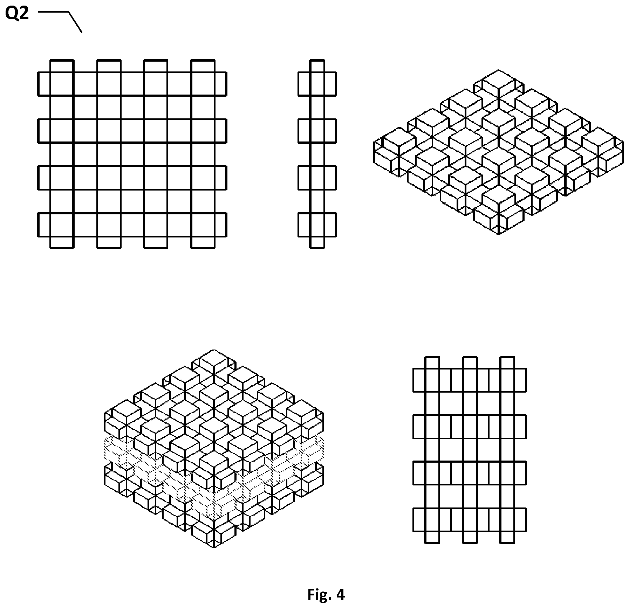

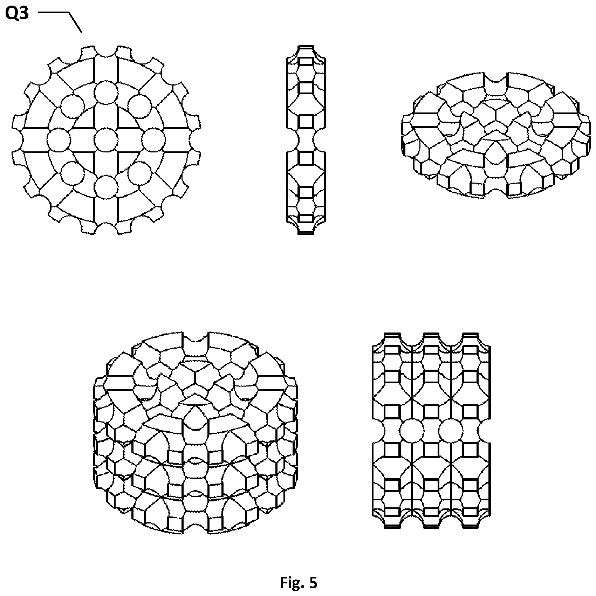

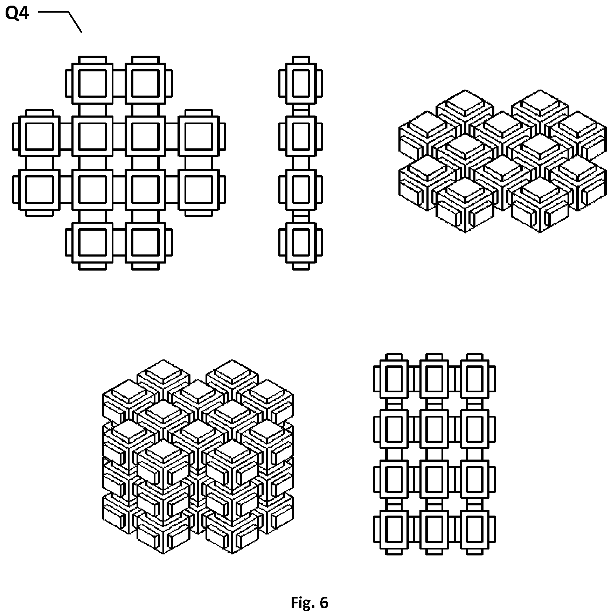

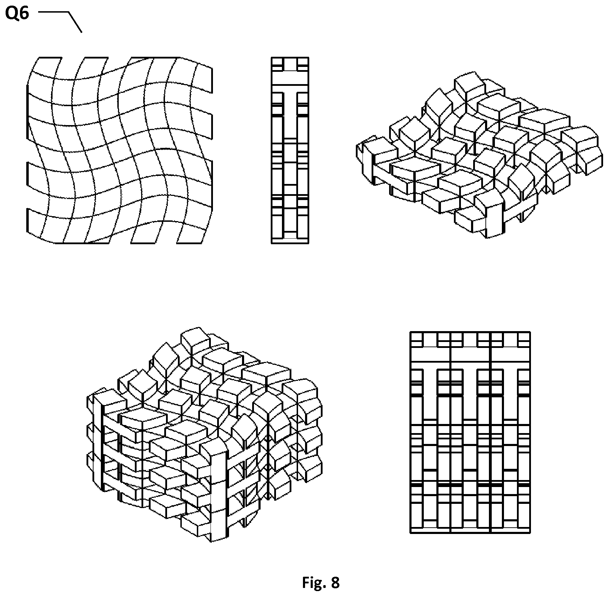

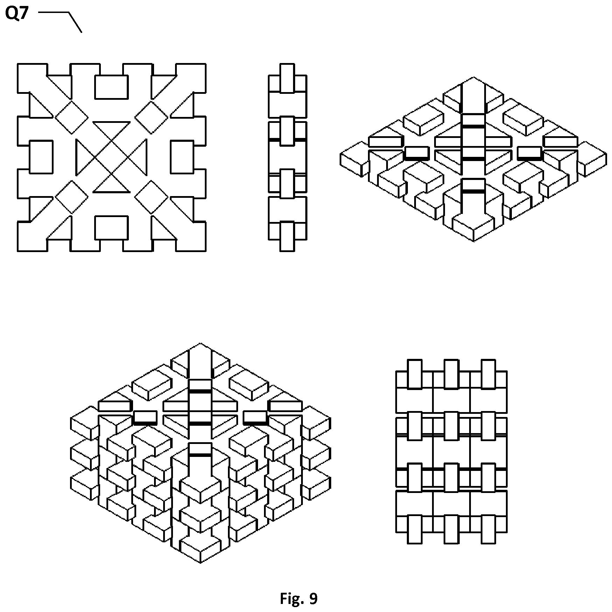

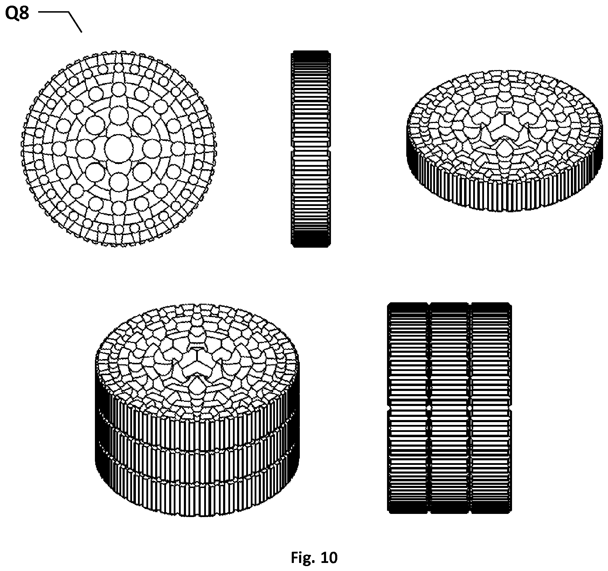

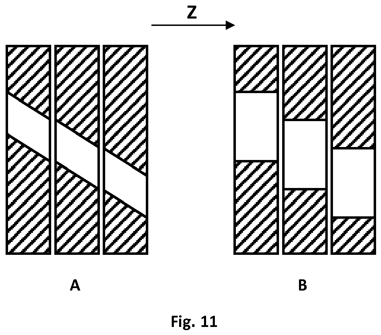

The channels, aligned with the Z-axis (Fig. 2 - Fig. 10), follow a straight path in some embodiments. However, the Z-axis channels or through-holes of the layer module can have up to 45º with the Z-axis (Fig. 11A), an angle advantageous for moulding, or even more in some embodiments. Alternatively, or additionally, each subsequent layer module can be slightly shifted (Fig. 11B) such that a similar effect is obtained of angled Z-axis channels. This shift can be translational (for example for the layer modules Q1 or Q2 of Fig. 3 - Fig. 4) or rotational (for example for the layer modules Q3 or Q8 of Fig. 5 or Fig. 10), or even both.

In some embodiments, when the layers are offset in any of the described approaches, the channels in both surfaces parallel to the XY plane, may have at least 10%, and up to 90% of the cross section overlapping between adjacent layers. In some embodiments, there will be a compromise - larger offsets between layers means the channels will be more tilted but, at the same time, there will be less continuity in the channels between subsequent layers and, conversely, the inverse.

The techniques suitable for obtaining the final product shapes can be any industrial technique that allows the joining and/or consolidation of materials, namely: Ultrasonic Welding; Rigid junctions (screws, spiking, rivets, etc...); Snap Fits; Sintering; Thermal fusion; or any other connection techniques suitable for the raw materials in use. It can be noted that minimizing the number of layer joints is relevant in order to minimize the probability of structural faults and layer adhesion issues. These shortcomings can be mitigated by reducing the number of layer joints or, also, by increasing the contact area being joined between layers.

In some embodiments, before or after performing the assembly operations, the parts may be submitted to a coating, thermal treatment and/or chemical treatment in order to improve functional and/or decorative performance.

The disclosure has advantages: a maximization of porosity with fully controlled channel sections; a controlled porosity by fully controlled channel sections; a modular solution allowing combinations of designs with different materials or even combinations of materials; easiness of mass production; low series cost due to the use of injection moulding technology; low complexity and low cost tools for the use in injection moulding technology. The use of this technology eliminates the need of using complementary operations of de-flashing, avoiding the possible quality problems like chipping.

Furthermore, by means of interchangeable moulding inserts, whereby the mould comprises an outer frame and interchangeable moulding inserts, the use of the same outer frame for the production of different modules geometries and versions is possible. This characteristic allows the reduction of the set-up costs.

Also, unique designs are feasible with improved design freedom, as designs previously not feasible in one step productions are possible to be achieved using this solution, namely designs with non-linear paths along the XY plan sections, or macro porous parts with interconnected channels in a totally closed outer contour.

The combination of different design versions allows porosity variations, as well as the achievement of tilted, even parabolic or spiral axis channels (made will several straight sections) along the Z-axis. There is also the possibility to obtain complex 3D shapes with non-straight, irregular or even randomly irregular inner channels, namely along the Z-axis. In some embodiments, the layer modules can simply be stacked in a random way. In some embodiments, the cross-sectional profile of the layer module z-channels is sufficiently wide that even if randomly stacked at least partial z-channels will form.

This production process provides stability in the moulding process in particular for complex geometries and thin walled products. General dimensions and shape of the final products can assume many possibilities, which may be subject to machining or other known material manipulation techniques, like drilling, milling, lathing, pressing.

The above described embodiments are combinable. The following dependent claims set out particular embodiments of the invention.

## Claims

1. Bone implant comprising a three-dimensional porous moulded-multilayer product (2) comprising a stack assembly of a plurality of injection moulded layer modules (1), said modules comprising:
a plurality of through-holes between the layer surfaces of said moulded layer module;
a plurality of longitudinal recesses on at least one of the layer surfaces of said moulded layer module, along at least two different directions, such that when assembled with a layer surface of another moulded layer module, longitudinal channels are formed;
wherein said through-holes and recess channels are interconnected.

2. Bone implant according to the previous claim, wherein the stack assembly of a plurality of moulded layer modules is obtainable by sintering.

3. Bone implant according to any of the previous claims comprising bioresorbable polymer, bioresorbable ceramic, bio-inert polymer, bio-inert ceramic, bio-inert metallic, or combinations thereof.

4. Bone implant according to any of the previous claims, wherein the plurality of modules is assembled with all layer faces of said modules positioned in the same direction.

5. Bone implant according to any one of the previous claims, wherein said module comprises alignment features on both layer surfaces of said moulded layer module, in particular protrusions and recesses for receiving protrusions, said features being such that, when a module is assembled with another module, a partial or full overlap of said through-holes is obtained.

6. Bone implant according to any one of the previous claims wherein the moulded-multilayer product is obtainable by powder injection moulding or thermoplastic injection moulding, comprising plastic, thermoplastic polymer, thermoset resin, metal, ceramic, or combinations thereof.

7. Bone implant according to any one of the previous claims wherein the cross-section of the through-holes and longitudinal channels of the moulded layer module is hexagonal, square, circular, and/or cross-shaped.

8. Bone implant according to any one of the previous claims wherein the through-holes of the moulded layer module are laid out in a grid, or concentrically, or both in a grid and concentrically.

9. Bone implant according to any one of the previous claims wherein the through-holes of the moulded layer module are perpendicular to the layer surfaces of said moulded layer module or are tilted relative to the layer surfaces of said moulded layer module.

10. Bone implant according to any of the previous claims wherein the assembled plurality of moulded layer modules is offset between adjacent moulded layer modules, in particular such that adjacent moulded layer modules are translationally, rotationally or randomly offset, in order that adjacent moulded layer modules through-holes are partially overlapping.

11. Method for producing a bone implant comprising a three-dimensional porous moulded-multilayer product (2) comprising the steps of:
injection moulding a plurality of moulded layer modules (1) of a bone implant
described in any of the claims 1-10;
assembling said plurality of moulded layer modules in a stack.

12. Method according to the previous claim wherein the moulding of the moulded layer module comprises powder injection moulding or thermoplastic injection moulding.

13. Method according to any of the claims 11 - 12 wherein the assembling of the moulded layer modules comprises sintering.

14. Method according to any of the claims 11 - 13 further comprising, in the moulding step of the moulded layer modules, moulding with a binder, and further comprising, before or during the assembly step, removing said binder, in particular thermally removing said binder during the assembly operation, said assembly operation being by sintering.

15. Method according to the previous claim where said binder is polymeric and the removal of said binder is thermal, catalytic or by solubility.

## Patentansprüche

1. Knochenimplantat mit einem porösen mehrschichtigen dreidimensionalen Formprodukt (2), das eine Stapelanordnung aus einer Vielzahl von im Spritzgussverfahren geformten Schichtmodulen (1) umfasst,
wobei die genannten Module umfassen:
eine Vielzahl von durchgehenden Öffnungen zwischen den Schichtoberflächen des genannten geformten Schichtmoduls;
eine Vielzahl von längsverlaufenden Vertiefungen auf mindestens einer der Schichtoberflächen des genannten geformten Schichtmoduls entlang mindestens zwei unterschiedliche Richtungen, so dass sich beim Zusammenfügen mit einer Schichtoberfläche eines anderen geformten Schichtmoduls Längskanäle bilden;
wobei die genannten durchgehenden Öffnungen und Vertiefungskanäle miteinander verbunden sind.

2. Knochenimplantat nach dem vorangehenden Anspruch, wobei sich die aus einer Vielzahl an geformten Schichtmodulen bestehende Stapelanordnung durch Sintern herstellen lässt.

3. Knochenimplantat nach einem der vorangehenden Ansprüche, umfassend bioresorbierbares Polymer, bioresorbierbare Keramik, bioinertes Polymer, bioinerte Keramik, bioinertes Metall oder Kombinationen davon.

4. Knochenimplantat nach einem der vorangehenden Ansprüche, wobei die Vielzahl der Module so angeordnet ist, dass alle Schichtflächen der genannten Module in die gleiche Richtung zeigen.

5. Knochenimplantat nach einem der vorangehenden Ansprüche, wobei das genannte Modul besondere Merkmale bei der Ausrichtung der beiden Schichtoberflächen des geformten Schichtmoduls aufweist, insbesondere Vorsprünge und Vertiefungen zur Aufnahme von Vorsprüngen, wobei die genannten Merkmale so gestaltet sind, dass beim Zusammensetzen eines Moduls mit einem anderen Modul eine teilweise oder vollständige Überlappung der durchgehenden Öffnungen erreicht wird.

6. Knochenimplantat nach einem der vorangehenden Ansprüche, wobei das mehrschichtige Formprodukt durch Pulverspritzgießen oder thermoplastisches Spritzgießen geformt wird, umfassend Kunststoff, thermoplastisches Polymer, duroplastisches Harz, Metall, Keramik oder Kombinationen davon.

7. Knochenimplantat nach einem der vorangehenden Ansprüche, wobei der Querschnitt der durchgehenden Öffnungen und Längskanäle des geformten Schichtmoduls sechseckig, quadratisch, rund und/oder kreuzförmig ist.

8. Knochenimplantat nach einem der vorangehenden Ansprüche, wobei die durchgehenden Öffnungen des geformten Schichtmoduls in einem Gitter oder konzentrisch oder in einem Gitter und konzentrisch angeordnet sind.

9. Knochenimplantat nach einem der vorangehenden Ansprüche, wobei die durchgehenden Öffnungen des geformten Schichtmoduls senkrecht zu den Schichtoberflächen des genannten geformten Schichtmoduls stehen oder relativ zu den Schichtoberflächen des genannten geformten Schichtmoduls geneigt sind.

10. Knochenimplantat nach einem der vorangehenden Ansprüche, wobei die zusammengesetzte Mehrzahl der geformten Schichtmodule zu den benachbarten geformten Schichtmodulen versetzt ist, insbesondere so, dass benachbarte geformte Schichtmodule verschoben, abwechselnd versetzt oder zufällig versetzt sind, so dass benachbarte durchgehenden Öffnungen der geformten Schichtmodule sich teilweise überlappen.

11. Verfahren zur Herstellung eines Knochenimplantats, umfassend ein poröses mehrschichtiges dreidimensionales Formprodukt (2), das folgende Schritte umfasst:
Spritzgießen einer Vielzahl von geformten Schichtmodulen (1) eines Knochenimplantats beschrieben in einem der Ansprüche 1-10;
Zusammensetzen der genannten Vielzahl von geformten Schichtmodulen zu einem Stapel.

12. Verfahren nach dem vorangehenden Anspruch, wobei das Formen des geformten Schichtmoduls Pulverspritzgießen oder Thermoplast-Spritzgießen umfasst.

13. Verfahren nach einem der Ansprüche 11-12, wobei das Zusammensetzen der geformten Schichtmodule Sintern umfasst.

14. Verfahren nach einem der Ansprüche 11-13, das beim Formen der geformten Schichtmodule ferner einen Schritt mit einem Bindemittel umfasst und ferner vor oder während des Montageschritts das Entfernen des genannten Bindemittels, insbesondere das thermische Entfernen des genannten Bindemittels während des Montagevorgangs, umfasst, wobei der genannte Montagevorgang durch Sintern erfolgt.

15. Verfahren nach dem vorangehenden Anspruch, wobei das genannte Bindemittel polymer ist und das Entfernen des genannten Bindemittels thermisch, katalytisch oder durch Löslichkeit erfolgt.

## Revendications

1. Implant osseux comprenant un produit multicouche moulé poreux en trois dimensions (2) comprenant un empillement d'une pluralité de modules à couche moulés par injection (1), lesdits modules comprenant:
une pluralité de trous traversant les surfaces de couche dudit module à couche moulé ;
une pluralité d'évidements longitudinaux sur au moins l'une des surfaces de couche dudit module à couche moulé, au long d'au moins deux différentes directions, de façon à ce qu'une fois qu'ils seront assemblés avec une surface de couche d'un autre module à couche moulé, des canaux longitudinaux se forment ;
dans lequel lesdits trous et canaux d'évidement sont interconnectés.

2. Implant osseux selon la revendication précédente, dans lequel l'empillement d'une pluralité de modules à couche moulés peut être obtenu par frittage.

3. Implant osseux selon l'une quelconque des revendications précédentes comprenant un polymère biorésorbable, une céramique biorésorbable, un polymère bio-inerte, une céramique bio-inerte, une pièce métallique bio-inerte, ou une combinaison de ceux-ci.

4. Implant osseux selon l'une quelconque des revendications précédentes, dans lequel la pluralité de modules est assemblée avec toutes les faces des couches desdits modules positionnées dans la même direction.

5. Implant osseux selon l'une quelconque des revendications précédentes, dans lequel ledit module comprend des caractéristiques d'allignement sur les deux surfaces de la couche dudit module à couche moulé, en particulier les saillies et évidements pour accueillir les saillies, lesdites caractéristiques étant telles que lorsqu'un module est assemblé avec un autre module, une superposition partielle ou totale desdits trous est obtenue.

6. Implant osseux selon l'une quelconque des revendications précédentes, dans lequel le produit multicouche moulé peut être obtenu par moulage par injection de poudre or par moulage par injection thermoplastique, comprenant du plastique, un polymère thermoplastique, une résine thermodurcissable, un métal, une céramique ou une combinaison de ceux-ci.

7. Implant osseux selon l'une quelconque des revendications précédentes, dans lequel la section transversale des trous et des canaux longitudinaux du module à couche moulé est héxagonale, carrée, circulaire et/ou transversale.

8. Implant osseux selon l'une quelconque des revendications précédentes, dans lequel les trous du module à couche moulé sont diposés en grille, ou de façon concentrique, ou en grille et de façon concentrique.

9. Implant osseux selon l'une quelconque des revendications précédentes, dans lequel les trous du module à couche moulé sont perpendiculaires aux surfaces de couche dudit module à couche moulé ou sont inclinés par rapport aux surfaces de couche dudit module à couche moulé.

10. Implant osseux selon l'une quelconque des revendications précédentes, dans lequel la pluralité assemblée de modules à couche moulés est compensée entre les modules à couche moulés, en particulier tels que les modules à couche moulés adjacents sont compensés de façon translationnelle, rotationnelle ou aléatoire, de façon à ce que les trous des modules à couche moulés adjacents se superposent partiellement.

11. Méthode pour produire un implant osseux comprenant un produit multicouche moulé poreux en trois dimensions (2) comprenant les étapes de :
moulage par injection d'une pluralité de modules à couche moulés (1) d'un implant osseux décrit dans l'une quelconque des revendications 1-10 ;
assemblage de ladite pluralité de modules à couche moulés en pile.

12. Méthode selon l'une quelconque des revendications précédentes, dans laquelle le moulage du module à couche moulé comprend un moulage par injection de poudre ou un moulage par injection thermoplastique.

13. Méthode selon l'une quelconque des revendications 11-12, dans laquelle l'assemblage des modules à couche moulés comprend un frittage.

14. Méthode selon l'une quelconque des revendications 11-13 comprenant également, durant l'étape de moulage des modules à couche moulés, un moulage avec un liant, et comprenant également, avant ou durant l'étape d'assemblage, l'élimination dudit liant, en particulier retirant ledit liant thermiquement durant l'opération d'assemblage, ladite opération d'assemblage étant faite par frittage.

15. Méthode selon l'une quelconque des revendications précédentes où ledit liant est polymérique et l'élimination dudit liant est thermique, catalytique ou par solubilité.
